# EUROPEAN PATENT APPLICATION

(11) **EP 2 484 283 A2**
(43) Date of publication of application: **08.08.2012**
(21) Application number: 12165583.1
(22) Date of filing: 23.11.2006
(51) Int. Cl.: A61B 5/145, G06K 7/00, H04B 1/38

(54) **Blood glucose meter capable of wireless communication**

(62) Divisional of application: 06818782.2
(71) Applicant: Lifescan Scotland Ltd, Inverness, IV2 3ED, Scotland (GB)
(72) Inventor: The designation of the inventor has not yet been filed
(74) Representative: UEXKÜLL & STOLBERG

(57) **Abstract**

The present invention relates to a blood glucose meter (200) comprising a blood glucose measuring module (234) for performing a blood glucose measuring function and a wireless communication module (236) adapted for establishing a wireless communication link with an external device and exchanging information with the external device via the wireless communication link according to a predetermined frequency format and protocol. The blood glucose measuring module (234) and the wireless communication module (236) are physically separate units electrically connected in order to allow for an exchange of electrical signals corresponding to information to be transmitted by the wireless communication module and/or information received by the wireless communication module (236). According to the invention, the blood glucose measuring module (234) and the wireless communication module (236) both comprise a connector component (238, 240), wherein the two connector components (238, 240) are adapted to releasably establish the electrical connection between the blood glucose measuring module (234) and the wireless communication module (236). The present invention further relates to a blood glucose system comprising such a blood glucose meter (200) and a number of additional wireless communication modules (236) adapted for different frequency formats and/or protocols, so that the frequency format and/or protocol according to which the blood glucose meter (200) can communicate with an external device can be changed by replacing the wireless communication module (236) with one of the additional wireless communication modules (236), and to a corresponding method of constructing a blood glucose meter (200).

## Description

The present invention relates to a blood glucose meter comprising a blood glucose measuring module for performing a blood glucose measuring function and a wireless communication module adapted for wireless communication with an external device, and to a method of constructing such a blood glucose meter.

Diabetes mellitus is a chronic metabolic disorder caused by an inability of the pancreas to produce sufficient amounts of the hormone insulin so that the metabolism is unable to provide for the proper absorption of sugar and starch. This failure leads to hyperglycemia, i.e. the presence of an excessive amount of glucose within the blood plasma. Persistent hyperglycemia causes a variety of serious symptoms and life threatening long term complications such as dehydration, ketoacidosis, diabetic coma, cardiovascular diseases, chronic renal failure, retinal damage and nerve damages with the risk of amputation of extremities. Because healing is not yet possible, a permanent therapy is necessary which provides constant glycemic control in order to always maintain the level of blood glucose within normal limits. Such glycemic control is achieved by regularly supplying external insulin to the body of the patient to thereby reduce the elevated levels of blood glucose.

External insulin was commonly administered by means of typically one or two injections of a mixture of rapid and intermediate acting insulin per day via a hypodermic syringe. While this treatment does not require the frequent estimation of blood glucose, it has been found that the degree of glycemic control achievable in this way is suboptimal because the delivery is unlike physiological insulin production, according to which insulin enters the bloodstream at a lower rate and over a more extended period of time. Improved glycemic control may be achieved by the so-called intensive insulinotherapy which is based on multiple daily injections, including one or two injections per day of long acting insulin for providing basal insulin and additional injections of rapidly acting insulin before each meal in an amount proportional to the size of the meal. Although traditional syringes have at least partly been replaced by insulin pens, the frequent injections are nevertheless very inconvenient for the patient. Substantial improvements in diabetes therapy have been achieved by the development of blood glucose systems relieving the patient of the daily use of syringes or insulin pens. Such blood glucose systems usually comprise a battery-operated blood glucose meter and one or more separate further devices, such as a battery-operated insulin pump. Further, such blood glucose systems usually comprise a control unit which is often provided as an integral part of the blood glucose meter, but may also be provided as a separate device communicating with the blood glucose meter and the remaining devices.

In such systems, the blood glucose meter is used to determine the blood glucose concentration, e.g. by receiving blood samples via enzyme-based test strips and calculating the blood glucose value based on the enzymatic reaction. Advantageously, the blood glucose system is configured such that the measured value is automatically delivered to the control unit. The insulin pump allows for the delivery of insulin in a more physiological manner and can be controlled to follow standard or individually modified protocols to give the patient a better glycemic control over the course of a day. It can be constructed as an implantable device for subcutaneous arrangement or can be constructed as an external device that is carried on the body of the patient.

The operation of the insulin pump and of other devices can be controlled and modified by means of the control unit. For example, delivery of suitable amounts of insulin by the insulin pump requires that the patient frequently determines his or her blood glucose level and inputs this value into the control unit, which then calculates a suitable modification to the default or currently in use insulin delivery protocol, i.e. dosage and timing, and subsequently communicates with the insulin pump to adjust its operation accordingly. In this regard, it may be necessary to use the control unit each time the patient eats to instruct the pump to administer a specified amount of insulin to cover that meal. Recently, a more or less closed-loop control has been realized in which the control unit modifies the insulin delivery protocol automatically.

In view of the permanence of the therapy, it is desirable to provide the diabetic patient with flexibility, convenience and ease of use in order to increase the quality of his or her life. In this regard, it is evident that cable connections between the individual devices of a blood glucose system are disadvantageous. Thus, it is known to provide a blood glucose meter with wireless communication capabilities.

In some of such meters, care has been taken to separate the part that is responsible for the measuring functionality yielding a metered value corresponding to blood glucose level from the part that is responsible for establishing a wireless communication link and receiving and/or transmitting information via this link. This arrangement has the advantage that in case changes to the wireless communication part are made upon further development of the blood glucose meter, such changes do not have an influence on the blood glucose measuring functionality. In this way, it is ensured that a modification which only relates to the wireless communication part does not have an impact on the critical medical functionality so that a new testing and validation of the blood glucose measuring part is avoided. In blood glucose meters, this issue is of great importance, because uncertainties with regard to the correct measurement of blood glucose level cannot be accepted.

One exemplary blood glucose meter constructed in this way is described in US 2003/0065536 A1. This reference discloses a blood glucose meter capable of communicating medically relevant data information to another device such as e.g. a central server or a mobile terminal. The blood glucose meter includes a blood glucose measuring part for executing a blood glucose measuring function and a communication part responsible for wireless communication according to e.g. radio frequency communication, infrared communication, the Bluetooth protocol and/or the TCP/IP protocol. Both parts are connected in such a manner that they can exchange data information according to a predetermined protocol under the control of the blood glucose measuring part. Otherwise, the functionalities of both parts are separated in order to obtain two physically and functionally separated parts for the above reasons. While this blood glucose meter may facilitate advancement to the next product cycle to some degree, it lacks desired flexibility for the meter to be easily adapted to operation in different environments. The communication device part has predetermined functions, and in case a particular application requires a deviating wireless approach, a complex modification procedure is necessary.

In a different field of medical devices, US 6,731,962 discloses a finger oximeter with remote telecommunication capabilities enabling the oximeter to transmit measurement data from a patient to a remote device. The oximetry circuitry and the transmission circuitry may be provided on a single or on two separate printed circuit boards. This device has the same disadvantages mentioned above for the known blood glucose meter.

It is the object of the present invention to provide a blood glucose meter providing a high degree of flexibility with regard to adapting it to operation in different wireless communication environments and that remedies the disadvantages found in the prior art, and to provide a blood glucose system including such a blood glucose meter as well as a method of constructing such a blood glucose meter.

This object is achieved by a blood glucose meter with the features of claim 1, by a blood glucose system with the features of claim 15, and by a method with the features of claim 17. Further preferred embodiments of the invention are the subject-matter of the respective dependent claims.

The blood glucose meter of the present invention comprises a blood glucose measuring module for performing a blood glucose measuring function, i.e. the module includes a means for performing such function, such as e.g. a means that is able to analyze blood samples on enzyme-based test strips, that can be inserted into a test strip receiving slot of the meter in order to determine the blood glucose level based on the enzymatic reaction. This blood glucose measuring module may be adapted to perform all steps necessary to measure, calculate and provide a value of blood glucose level. For some applications, however, it may be advantageous if the blood glucose measuring module is adapted to perform only a part of these steps, while one or more additional devices, connected to the blood glucose meter by means of e.g. a wireless or cable connection, are provided to perform the remaining steps. For example, an external sensor may be provided for transmitting a signal characteristic of blood glucose level to the blood glucose meter, from which signal the blood glucose meter calculates and displays the blood glucose level.

The blood glucose meter further comprises a wireless communication module adapted for establishing a wireless communication link with an external device and exchanging information with the external device via the wireless communication link according to a predetermined frequency format and protocol. In the context of the present invention, the term frequency format refers to the physical characteristics of the wireless signals such as frequency or modulation. Although the wireless communication link will usually be bidirectional, for simple applications the link can also be unidirectional, i.e. the wireless communication module may be configured to only transmit or to only receive messages to or from the external device.

The blood glucose measuring module and the wireless communication module are physically separate units. In the context of the present invention, this means that the two modules as a whole are distinct structural parts which may be completely spatially separated from each other. However, they are releasably electrically connected in order to allow for an exchange of electrical signals corresponding to information to be transmitted by the wireless communication module and/or information received by the wireless communication module, i.e. in accordance with the considerations mentioned above the exchange of signals may be bidirectional or unidirectional.

It is to be noted that in addition to a means for performing a blood glucose measuring function, the blood glucose measuring module may also include other means for performing other functions relevant to the operation of the blood glucose meter. Only means relating to the wireless communication are necessarily disposed separate from this module and are located in the wireless communication module. In case the blood glucose measuring module includes a substantial part of the functional means or substantially all functional means of the blood glucose meter, the blood glucose measuring module could also be referred to as the main module of the meter.

According to the present invention, the blood glucose measuring module and the wireless communication module both comprise a connector component adapted to releasably establish the electrical connection between the blood glucose measuring module and the wireless communication module. Thus, the connector component of the blood glucose measuring module and the connector component of the wireless communication module can cooperate to effect the electrical connection between the two modules. This electrical connection is releasable, i.e. it can be selectively and repeatedly disconnected and re-established.

This construction provides the advantage that any connection and functional cooperation between the two physically separate modules can be easily and readily broken in case it is desired to replace one of the modules with another similar module providing a different functionality. Thus, while the above-referenced advantages due to separation of the blood glucose measuring functionality and the wireless communication functionality into two distinct units, i.e. the functional independence of this units, found in prior art blood glucose meters are retained, it is very simple to quickly replace a first wireless communication module, which is e.g. adapted for communication according to the Bluetooth standard, with a second wireless communication module, which is e.g. adapted for communication according to the ZigBee or ISM standard, thereby adapting the blood glucose meter to a different wireless communication environment. This advantageous flexibility can be utilized at the manufacturing stage or even later-on by the user. Thus, advantageously the blood glucose meter is constructed such that the frequency format and/or protocol according to which the blood glucose meter can communicate with an external device can be changed by replacing the wireless communication module with another wireless communication module which can be connected to the connector component of the blood glucose measuring module by means of its own connector component and which is adapted to communicate according to a different frequency format and/or protocol.

Moreover, the construction according to the invention provides further advantages during the quality control procedure. Because the electrical connection between the blood glucose measuring module and the wireless communication module is releasable and the blood glucose measuring functionality and the wireless communication functionality are compartmentalized in different modules, separate quality control tests can be performed on the modules after spatially separating the modules. This avoids problems and a complicated procedure due to the two modules being electrically connected and in close proximity to each other. Otherwise, problems could arise e.g. due to the wireless signal generated by the wireless communication module interfering with the quality control testing of the blood glucose measuring module. Thus, the likelihood of encountering interferences during quality control testing can be reduced by performing the two quality control tests entirely separately in such a manner that the blood glucose measuring module and the wireless communication module are not electrically connected and not in close proximity to each other.

In an advantageous version of the invention, the blood glucose meter is constructed such that the frequency format and/or protocol according to which the blood glucose meter can communicate with an external device can be changed solely by replacing the wireless communication module with another wireless communication module which is adapted to communicate according to a different frequency format and/or protocol. Thus, in order to adapt the meter to a different wireless communication environment, no other steps but disconnecting the present wireless communication module from the blood glucose measuring module, removing the wireless communication module and connecting a different wireless communication module having the desired wireless communication capabilities to the blood glucose measuring module are necessary for the adaptation. In particular, no changes whatsoever have to be made to the blood glucose measuring module. For this purpose, the electrical signals provided and expected by the blood glucose measuring module at its connector component are predetermined, and the new wireless communication module has to operate in accordance with the standard constituted by the corresponding set of predetermined signals.

In a preferred embodiment of the blood glucose meter, the blood glucose measuring module comprises a control means operable to control the electrical signals provided by the blood glucose measuring module at its connector component.

It is further preferred if the wireless communication module comprises a memory chip connector for electrically connecting the wireless communication module with a non-volatile semi-conductor memory card or a so-called smart card, wherein the control means of the blood glucose measuring module is adapted to operate in accordance with information stored in a non-volatile semiconductor memory card or smart card connected to the memory chip connector of the wireless communication module. In this regard, it is also possible that the memory card or smart card includes circuitry which is connected via the memory chip connector and the connector components of the two modules to the control means of the blood glucose measuring device and then forms part of the control means to thereby change or adapt the operation of the control means to the particular wireless communication module. In any case, the provision of a memory chip connector results in increased flexibility because the signals provided and expected by the blood glucose measuring module at its connector may be modified to thereby improve the capabilities of the wireless communication module and the meter as a whole.

In the alternative, it is preferred if the control means can be reprogrammed in order to change the electrical signals provided and/or expected during operation by the blood glucose measuring module at its connector component, i.e. the electrical signals can be reprogrammed in their functions. In a preferred embodiment such reprogramming can be effected by the wireless communication module. For this purpose, the wireless communication module may comprise a memory chip connector for electrically connecting the wireless communication module with a non-volatile semi-conductor memory card or smart card, wherein the control means of the blood glucose measuring module can be reprogrammed in accordance with information stored in a non-volatile semiconductor memory card or smart card connected to the memory chip connector of the wireless communication module. For example, the meter may be constructed such that this reprogramming is effected automatically upon establishing the releasable electrical connection between the blood glucose measuring module and the wireless communication module or by inserting a memory card or smart card into the memory chip connector. This design provides additional flexibility because the memory card or smart card does not always have to be present in the wireless communication module. In any case, the frequency format and/or protocol according to which the blood glucose meter can communicate with an external device can advantageously be changed by replacing the wireless communication module with another wireless communication module which is adapted to communicate according to a different frequency format and/or protocol and suitably reprogramming the control means of the blood glucose measuring module.

It should be noted that each of the memory chip connectors mentioned above could also be provided not in the wireless communication module, but at a different location in the blood glucose meter of the present invention. For example, they could be provided in the blood glucose measuring module. In any case, in the assembled state of the blood glucose meter the memory chip connectors have to be connected to the control means of the blood glucose measuring module. However, in most cases, disposing the memory chip connector in the wireless communication module provides the advantages of particular flexibility and space economy within the blood glucose measuring module.

It is preferred that the wireless communication module comprises a memory chip connector for electrically connecting the wireless communication module with a non-volatile semi-conductor memory card. This memory chip connector can be one of the memory chip connectors mentioned above or an additional memory chip connector. In this way a variety of different functions of the wireless communication module and/or the blood glucose measuring module may be modified and adapted in a particularly simple manner. Further, a memory card or smart card can also serve as a storage means for storing various information relating to the measurement results obtained by the meter. In addition or in the alternative, one or more memory chip connectors for connecting a non-volatile semiconductor memory card with the blood glucose meter and fulfilling one or more of the above or one or more other functions could also be provided at other locations in the blood glucose meter of the present invention, e.g. in the blood glucose measuring module. For any memory chip connector present in the wireless communication module (or at a different location), it is advantageous if the memory chip connector is orientated such that a set of contacts on a memory card connected to the memory chip connector face downwards allowing a user to more easily observe upward facing artwork on the memory card as it is inserted into said blood glucose meter. In this way, the identity of the card can be easily verified without a need to remove the card from the memory chip connector.

According to a preferred embodiment of the invention, the wireless communication module does not comprise an own power supply. Rather, a corresponding power supply for operating the wireless communication module is disposed in the blood glucose measuring module. This power supply can be the power supply of the blood glucose measuring module or a separate, dedicated power supply. In any case, the electrical signals provided during operation by the blood glucose measuring module at its connector component include signals for supplying power to the wireless communication module. This construction provides the advantage of the possibility of a particularly simple and low-cost design of the wireless communication module, which is highly advantageous in view of the wireless communication modules being envisaged for interchangeability.

It is preferred that the wireless communication module comprises a transceiver, an antenna impedance matching network and an antenna, and possibly also an electrostatic discharge (ESD) protection circuit. In this manner, the wireless communication module is a self-contained unit. Arrangement of the antenna in close proximity to the remaining components of the wireless communication module is highly advantageous.

It is further preferred that the blood glucose meter comprises a housing in which the blood glucose measuring module and the wireless communication module are enclosed to provide a meter which is compact and easily manageable for the user.

In a preferred embodiment of the invention, the blood glucose measuring module comprises or consists of a first printed circuit board on which blood glucose measuring circuitry - and possibly further circuitry if the first printed circuit board is the main board of the meter - is mounted or provided, and the wireless communication module comprises or consists of a second printed circuit board, different from the first printed circuit board, on which wireless communication circuitry is mounted or provided. In this case, it is advantageous if the wireless communication module comprises a transceiver, an antenna impedance matching network and an antenna as well as possibly an ESD protection circuit, and if all these elements of the wireless communication module are mounted or provided on the second printed circuit board. Further, a particularly simple and efficient design can be realized if the first printed circuit board is a motherboard and the second printed circuit board is a daughterboard. Further, it is preferred if the second printed circuit board is disposed on the first printed circuit board. In any case, it is an advantage of this construction that the second printed circuit board or daughter board helps to economize space on the first printed circuit board or mother board.

It is preferred if one of the connector components of the blood glucose measuring module and the wireless communication module is the plug component of a plug and socket connector and the other connector component is the socket component of the plug and socket connection. Such a plug and socket connection is particularly easy to operate and simple in construction. The releasable electrical connection may be established by directly connecting the plug and the socket, i.e. by inserting the plug on the wireless communication module or the blood glucose measuring module into the socket on the blood glucose measuring module or wireless communication module, respectively, or by connecting them via an adapter or a cable. The connector components of the blood glucose measuring module and the wireless communication module may also be adapted to be connected by a cable having suitable connector components at its ends. Such a construction provides flexibility with regard to the physical arrangement of the two modules within the meter.

The wireless communication module may advantageously be adapted to communicate according to the ISM, Bluetooth, ZigBee or WLAN standard or even according to two or more of these standards.

The blood glucose meter of the invention described above may advantageously be part of a blood glucose meter system further comprising at least one further wireless communication module similar to the wireless communication module but adapted for establishing a wireless communication link with an external device and exchanging information with the external device via the wireless communication link according to a predetermined frequency format and protocol different from the frequency format and/or protocol of the wireless communication module. As is evident from the above description, with such a system the frequency format and/or protocol according to which the blood glucose meter can communicate with an external device can be changed by replacing the wireless communication module with one of the at least one further wireless communication module by establishing a releasable connection between the blood glucose measuring module and the new wireless communication module by means of their connector components. Of course, such a system can also be regarded as comprising a blood glucose meter as described above with or without a wireless communication module connected to the blood glucose measuring module as well as a number or set of wireless communication modules which are adapted for different wireless communication capabilities. This set of wireless communication modules may e.g. include wireless communication modules adapted to communicate according to the ISM, Bluetooth, ZigBee or WLAN standard or even according to two or more of these standards.

Any of the blood glucose meters of the present invention described above may advantageously be constructed by a method in which a blood glucose measuring module for performing a blood glucose measuring function is provided and a plurality of wireless communication modules are provided, each adapted for establishing a wireless communication link with an external device and exchanging information with the external device via the wireless communication link according to a predetermined frequency format and protocol different from the frequency format and/or protocol of the other wireless communication modules. As noted in detail above, the blood glucose measuring modules and the wireless communication module are physically separate units and the blood glucose measuring module and each wireless communication module comprise respective connector components by means of which a selected one of the wireless communication modules may be releasably electrically connected in order to allow for an exchange of electrical signals corresponding to information to be transmitted by the respective wireless communication module and/or information received by the respective wireless communication module. Subsequently, one of the plurality of wireless communication modules is selected in accordance with the desired frequency format and protocol, and an electrical connection between the selected wireless communication module and the blood glucose measuring module is established by means of the respective connector components to thereby form the blood glucose meter. By this method, a manufacturer or even a user may easily and in a flexibly manner construct a blood glucose meter tailored to a particular application.

Further, the following constitute preferred embodiments of the invention:
Embodiment 1: A blood glucose meter (200) comprising a blood glucose measuring module (234) for performing a blood glucose measuring function and a wireless communication module (236) adapted for establishing a wireless communication link with an external device and exchanging information with the external device via the wireless communication link according to a predetermined frequency format and protocol, wherein the blood glucose measuring module (234) and the wireless communication module (236) are physically separate units electrically connected in order to allow for an exchange of electrical signals corresponding to information to be transmitted by the wireless communication module (236) and/or information received by the wireless communication module (236), characterized in that the blood glucose measuring module (234) and the wireless communication module (236) both comprise a connector component (238, 240), wherein the two connector components (238, 240) are adapted to releasably establish the electrical connection between the blood glucose measuring module (234) and the wireless communication module (236).
Embodiment 2: The blood glucose meter according to embodiment 1, wherein the frequency format and/or protocol according to which the blood glucose meter (200) can communicate with an external device can be changed by replacing the wireless communication module (236) with another wireless communication module (236) which is adapted to communicate according to a different frequency format and/or protocol.
Embodiment 3: The blood glucose meter according to embodiment 1 or embodiment 2, wherein the blood glucose measuring module (234) comprises a control means (228) operable to control the electrical signals provided by the blood glucose measuring module (234) at its connector component (238).
Embodiment 4: The blood glucose meter according to any of embodiments 1 to 3, wherein the frequency format and/or protocol according to which the blood glucose meter (200) can communicate with an external device can be changed solely by replacing the wireless communication module (236) with another wireless communication module (236) which is adapted to communicate according to a different frequency format and/or protocol.
Embodiment 5: The blood glucose meter according to embodiment 3, wherein the wireless communication module (236) comprises a memory chip connector (232) for electrically connecting the wireless communication module (236) with a non-volatile semi-conductor memory card (290), and wherein the control means (228) of the blood glucose measuring module (234) is adapted to operate in accordance with information stored in a non-volatile semiconductor memory card (290) connected to the memory chip connector (232) of the wireless communication module (236).
Embodiment 6: The blood glucose meter according to embodiment 3, wherein the control means (228) can be reprogrammed in order to change the electrical signals provided during operation by the blood glucose measuring module (234) at its connector component (238).
Embodiment 7: The blood glucose meter according to embodiment 6, wherein the wireless communication module (236) comprises a memory chip connector (232) for electrically connecting the wireless communication module (236) with a non-volatile semi-conductor memory card (290), and wherein the control means (228) of the blood glucose measuring module (234) can be reprogrammed in accordance with information stored in a non-volatile semiconductor memory card (290) connected to the memory chip connector (232) of the wireless communication module (236).
Embodiment 8: The blood glucose meter according to embodiment 6 or embodiment 7, wherein the frequency format and/or protocol according to which the blood glucose meter (200) can communicate with an external device can be changed by replacing the wireless communication module (236) with another wireless communication module (236) which is adapted to communicate according to a different frequency format and/or protocol and reprogramming the control means (228) of the blood glucose measuring module (234).
Embodiment 9: The blood glucose meter according to any of embodiments 1 to 8, wherein the wireless communication module (236) comprises a memory chip connector (232) for electrically connecting the wireless communication module (236) with a non-volatile semi-conductor memory card (290).
Embodiment 10: The blood glucose meter according to any of embodiments 5, 7, and 9, wherein the memory chip connector (232) is orientated such that a set of contacts (292) on a memory card (290) connected to the memory chip connector (232) face downwards allowing a user to more easily observe upward facing artwork on the memory card (290) as it is inserted into said blood glucose meter (200).
Embodiment 11: The blood glucose meter according to any of embodiments 1 to 10, wherein the blood glucose measuring module (234) comprises a power supply for operating the wireless communication module (236), and wherein the electrical signals provided during operation by the blood glucose measuring module (234) at its connector component (238) include signals for supplying power to the wireless communication module (236).
Embodiment 12: The blood glucose meter according to any of embodiments 1 to 11, wherein the wireless communication module (236) comprises a transceiver (218), an antenna impedance matching network (21) and an antenna (246, 248).
Embodiment 13: The blood glucose meter according to embodiment 12, wherein the wireless communication module (236) further includes an electrostatic discharge (ESD) protection circuit (244). Embodiment 14: The blood glucose meter according to any of embodiments 1 to 13, wherein the blood glucose meter (200) comprises a housing (201, 207) in which the blood glucose measuring module (234) and the wireless communication module (236) are enclosed.
Embodiment 15: The blood glucose meter according to any of embodiments 1 to 14, wherein the blood glucose measuring module (234) comprises a first printed circuit board on which blood glucose measuring circuitry (220) is mounted, and the wireless communication module (236) comprises a second printed circuit board on which wireless communication circuitry (218, 219, 246) is mounted.
Embodiment 16: The blood glucose meter according to embodiment 12 and 15, wherein the transceiver (218), the antenna impedance matching network (219) and the antenna (246, 248) are mounted on the second printed circuit board.
Embodiment 17: The blood glucose meter according to embodiment 13 and 16, wherein the ESD protection circuit (244) is mounted on the second printed circuit board.
Embodiment 18: The blood glucose meter according to any of embodiments 15 to 17, wherein the first printed circuit board is a motherboard and the second printed circuit board is a daughterboard.
Embodiment 19: The blood glucose meter according to any of embodiments 15 to 18, wherein the second printed circuit board is disposed on the first printed circuit board.
Embodiment 20: The blood glucose meter according to any of embodiments 1 to 19, wherein one of the connector components (238, 240) of the blood glucose measuring module (234) and the wireless communication module (236) is the plug component of a plug and socket connector and the other connector component (238, 240) is the socket component of the plug and socket connection.
Embodiment 21: The blood glucose meter according to any of embodiments 1 to 19, wherein the connector components (238, 240) of the blood glucose measuring module (234) and the wireless communication module (236) are adapted to be connected by a cable having suitable connector components at its ends.
Embodiment 22: The blood glucose meter according to any of embodiments 1 to 21, wherein the wireless communication module (236) is adapted to communicate according to the ISM, Bluetooth, ZigBee or WLAN standard.
Embodiment 23: A blood glucose meter system comprising a blood glucose meter (200) according to any of embodiments 1 to 19, and at least one further wireless communication module (236) similar to the wireless communication module (236) but adapted for establishing a wireless communication link with an external device and exchanging information with the external device via the wireless communication link according to a predetermined frequency format and protocol different from the frequency format and/or protocol of the wireless communication module (236), wherein the frequency format and/or protocol according to which the blood glucose meter (200) can communicate with an external device can be changed by replacing the wireless communication module (236) with one of the at least one further wireless communication module (236).
Embodiment 24: The blood glucose meter system according to embodiment 23, wherein the wireless communication module (236) and the at least one further wireless communication module (236) include wireless communication modules (236) adapted to communicate according to the ISM, Bluetooth, ZigBee or WLAN standard.
Embodiment 25: A method of constructing a blood glucose meter (200) according to any of embodiments 1 to 22 comprising the steps of providing a blood glucose measuring module (234) for performing a blood glucose measuring function, providing a plurality of wireless communication modules (236), each adapted for establishing a wireless communication link with an external device and exchanging information with the external device via the wireless communication link according to a predetermined frequency format and protocol different from the frequency format and/or protocol of the other wireless communication modules (236), wherein the blood glucose measuring module (234) and the wireless communication modules (236) are physically separate units and wherein the blood glucose measuring module (234) and each wireless communication module (236) comprise respective connector components (238, 240) by means of which a selected one of the wireless communication modules (236) may be releasably electrically connected to the blood glucose measuring module (234) in order to allow for an exchange of electrical signals corresponding to information to be transmitted by the respective wireless communication module (236) and/or information received by the respective wireless communication module (236), selecting one of the plurality of wireless communication modules (236) in accordance with the desired frequency format and protocol, and establishing an electrical connection between the selected wireless communication module (236) and the blood glucose measuring module (234) by means of the respective connector components (238, 240).

In the following, the invention is explained in more detail for preferred embodiments with reference to the figures.
- Figure 1: is a top perspective view of a blood glucose meter according to the present invention.
- Figure 2: is a schematic diagram showing the blood glucose meter of Figure 1 wirelessly communicating with a plurality of external medical devices.
- Figure 3: is a simplified bottom plan view of a motherboard of the blood glucose meter shown in Figure 1.
- Figure 4: is a simplified top plan view of the motherboard shown in Figure 3.
- Figure 5: is a simplified top plan view of a daughterboard of the blood glucose meter shown in Figure 1.
- Figure 6: is a simplified bottom plan view of the daughterboard shown in Figure 5.
- Figure 7: is a bottom plan view of a memory chip for use with the daughterboard shown in Figures 5 and 6.
- Figure 8: is a top plan view of the memory chip shown in Figure 7.
- Figure 9: is a simplified bottom perspective view of the daughterboard shown in Figures 5 and 6.
- Figure 10: is a simplified bottom perspective view of the blood glucose meter with a bottom housing half removed.

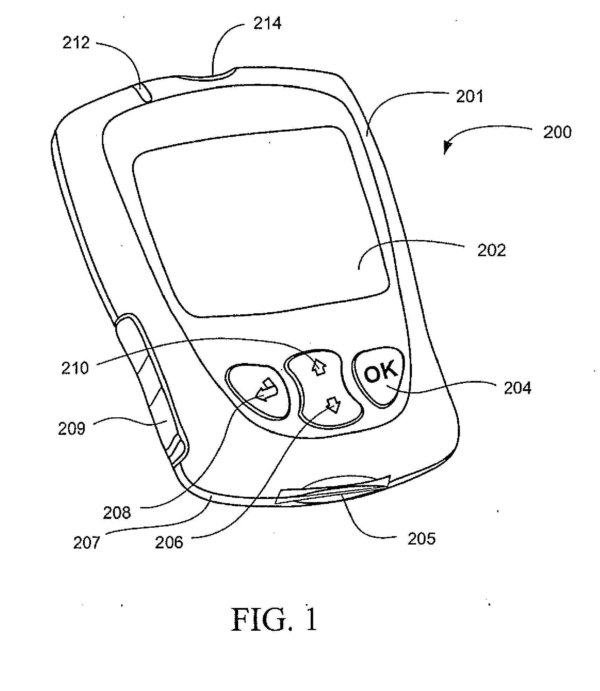

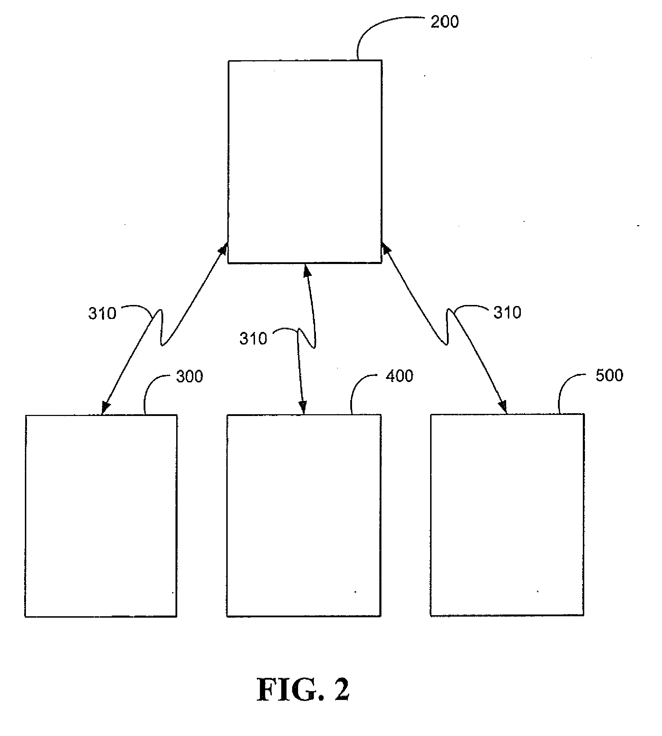

Figure 1 is a perspective view of a blood glucose measurement device or meter in accordance with the present invention, which in a particular embodiment of the invention is provided in the form of a remote controller 200. This remote controller 200 may e.g. be part of the blood glucose system schematically shown in Figure 2. A medical device such as, for example, this glucose measurement device 200 may have the ability to wirelessly communicate with one or more peripheral medical devices. In the embodiment of this invention shown in Figure 2, the blood glucose measurement device or remote controller 200 communicates via a wireless signal 310 with peripheral medical devices such as, for example, an insulin pump 300 for dispensing insulin to the blood circuit of a patient, a continuous glucose monitor (CGM) 400, an insulin pen 500, or a combination thereof. The insulin pump 300 may be an external device to be worn on the body of a patient, or it may be constructed as an implantable device to be disposed subcutaneously.

Recently, there has been a continuing development in the field of wireless communications and it is likely that these improvements will continue in the future. There will also likely be a need for additional types of peripheral medical devices to wirelessly communicate with a blood glucose measurement device for facilitating the therapy of people with diabetes. This has led to a need for designing a medical device such as a blood glucose meter which can have its hardware easily adapted to a new wireless communication environment or application or upgraded by using a modular architecture. In an embodiment of this invention, the wireless circuitry is compartmentalized from the blood glucose measuring circuitry. Thus, in such a design, only the wireless circuitry must be replaced while the blood glucose measuring circuitry will require little or no modification. In general, this will make adapting, changing or upgrading the wireless functionality of a blood glucose measurement device faster and easier to perform.

Remote controller 200 may be used to episodically measure blood glucose and to wirelessly control insulin pump 300 and/or other peripheral medical device(s). In an embodiment of this invention, remote controller 200 may be a master and insulin pump 300 and/or other peripheral medical device(s) may be a slave. As shown in Figure 1, remote controller 200 includes a first housing half 201, a display 202, an OK button 204, a universal port connector 205, a down button 206, a second housing half 207, a back button 208, a port cover 209, an up button 210, light emitting diode (LED) 212, and a strip port connector (SPC) 214. In an embodiment of this invention, a first housing half 201 and second housing half 207 may adapt to join together to form an ergonomically shaped handheld glucose meter which encloses both a motherboard and a daughterboard which will be described in detail below.

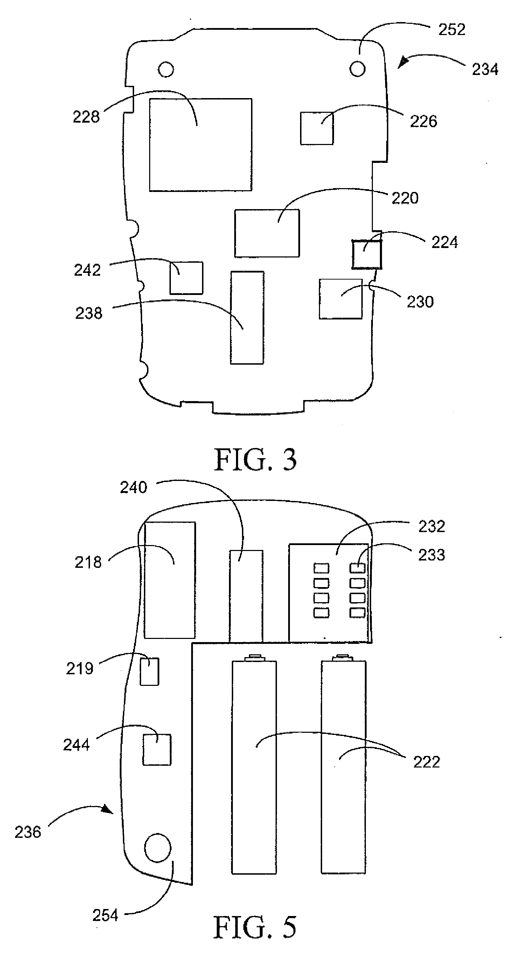

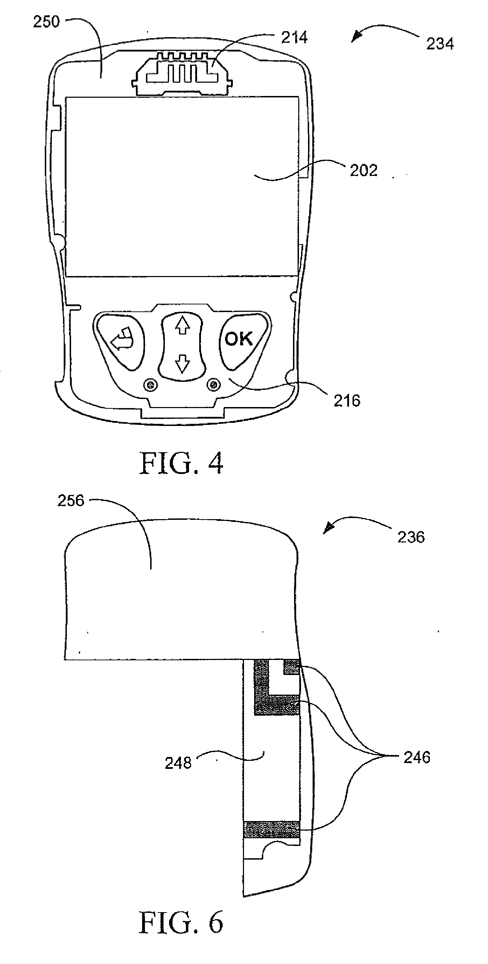

Figures 3 and 4 show a simplified schematic bottom and top plan view, respectively, of an embodiment of a motherboard 234 having a bottom surface 252 and a top surface 250. As shown in Figure 4, motherboard 234 has mounted on top surface 250, the strip port connector 214, the display 202, and a plurality of navigation buttons 216, namely the OK button 204, the down button 206, the back button 208 and the up button 210 (see

Figure 1) for effecting user entries e.g. by navigating through a menu structure of a user interface. The navigation buttons 216 may be utilized for initiating a particular action by the remote controller 200 or to input data into the remote controller 200, e.g. in order to adjust the operation of the insulin pump 300 with regard to various patient parameters such as e.g. his or her weight. As shown in Figure 3, motherboard 234 has mounted on bottom surface 252 an alarm 226, a blood glucose measurement means 220 for determining the level of blood glucose, a wired communication port 224, a memory 230, a first connector 238, a motherboard electrostatic discharge (ESD) circuitry 242, and a microprocessor 228.

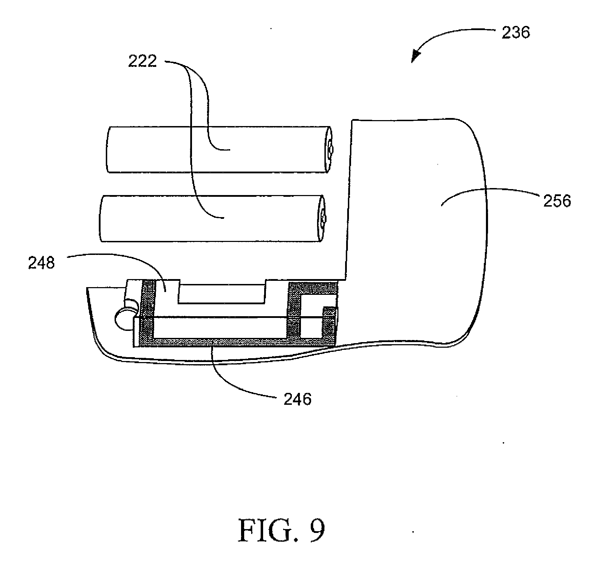

Figures 5 and 6 show a simplified schematic top and bottom plan view, respectively, of an embodiment of a daughterboard 236 having a top surface 254 and a bottom surface 256. As shown in Figure 5, daughterboard 236 has mounted on its top surface 254, a memory chip port 232, a daughterboard ESD circuitry 244, an antenna impedance matching network 219, a radio frequency transceiver 218, and a second connector 240. As shown in Figure 6, daughterboard 236 has mounted on its bottom surface 256, a dielectric material 248 and an antenna element 246 coupled to the transceiver 218. It should be noted that a combination of dielectric material 248 and antenna element 246 may be simply referred to as an antenna. Antenna element 246 may be a contiguous conductive material having a 3-dimensional shape which is disposed on a portion of dielectric material 248 as also shown in Figure 9. The use of daughterboard 236 provides an additional set of two surfaces (i.e. top surface 254 and bottom surface 256) in which electronic components can be mounted. This allows more electronics to be efficiently enclosed within first housing half 201 and second housing half 207. The daughterboard 236 may also include control electronics, e.g. in the form of a microprocessor, operable to compose data frames to be transmitted by means of the transceiver 218, to analyze data frames received by the transceiver 218, and to activate and deactivate the transceiver 218 to transmit and/or receive data frames.

Motherboard 234 includes all of the functional circuitry required for performing an episodic glucose measurement test, whereas daughterboard 236 includes all of the functional circuitry required for a wireless communication with one or more peripheral medical devices such as insulin pump 300. From the above description, it is evident that the motherboard 234 forms a blood glucose measuring module in accordance with the present invention and that the daughterboard 236 forms a wireless communication module in accordance with the present invention. In the shown embodiment, motherboard 234 and daughterboard 236 are electrically connected, respectively, by the first connector 238 and the second connector 240, both of which are adapted to reversibly mate and to effect a releasable electrical connection which can be repeatedly and selectively disconnected and re-established. In an embodiment of this invention, motherboard 234 and daughterboard 236 are disposed in a stacking orientation such that when they are mated together by means of connectors 238 and 240 bottom surface 252 of motherboard 234 is orientated in a spaced apart and facing arrangement with top surface 254 of daughterboard 236 (see Figures 3, 5, and 10).

Figure 9 shows two batteries 222 which are disposed in a position such that a major axis of antenna element 246 is parallel to a major axis of batteries 222. Batteries 222 may be disposed in a battery compartment which is integrated as a portion of second housing half 207 (not shown). In the embodiment shown in Figures 5, 6 and 9, daughterboard 236 has an "L" shape to provide room for batteries 222 which in this case are two AAA alkaline batteries as shown in Figures 5 and 9.

Remote controller 200 may be designed such that it can be operated while on a tabletop or while being held by a user's hand. First housing half 201 may be a top surface of remote controller 200 and second housing half 207 may be a bottom surface of remote controller 200. During the use of remote controller 200, its bottom surface should be disposed on either a tabletop or the user's hand and thus should not be viewable by a user. However, the top surface of remote controller 200 should be viewable by a user during use. Therefore, display 202 and navigation buttons 216 should be mounted on top surface 250 of motherboard 234. In addition, top surface 250 of motherboard 234 should be disposed immediately underneath first housing half 201 such that corresponding apertures within first housing half 201 adapt to the shape of display 202 and navigation buttons 216. This allows display 202 and navigation buttons 216 to be viewable on the top surface of remote controller 200.

It is desirable to make the size of remote controller 200 relatively small so that it can easily fit into a palm of a user's hand. In general, a length and a width of motherboard 234 must be about the same as remote controller 200 in order to efficiently use all of the usable space available within remote controller 200 for mounting electronic components. Typically, there is a desire to have sufficiently large navigation buttons 216 and display 202 so that a user can easily see and handle them. Therefore, in order to design remote controller 200 as small as possible and have sufficiently large navigation buttons 216 and display 202, motherboard 234 should have an efficiently packed set of electronic components leaving little to no usable space. In an embodiment of this invention, the set of electronic components mounted to top surface 250 on motherboard 234 may include display 202, navigation buttons 216, and strip port connector 214. Figure 4 shows that the set of electronic components occupy nearly all of the usable area of the top surface of motherboard 234 making it difficult to mount additional electronic components without increasing the size of motherboard 234.

Display 202 may be a liquid crystal display (LCD) to show both textual and graphical information to a user. A user interface (UI) may be a software driven menu that is shown on display 202 that enables the user to operate remote controller 200. A user can navigate through the UI using navigation buttons 216 which include up button 210, down button 206, OK button 204, and back button 208. In an embodiment of this invention, the UI allows a user to operate an insulin pump, query the status of the insulin pump, measure blood glucose episodically, and to display data on display 202 (e.g. glucose concentration versus time).

Alarm 226 which may be in a variety of forms to warn a user of various statuses that might need an actionable response. For example, alarm 226 may include an audio alarm (monophonic beeps or polyphonic tones), a vibratory alarm, or a LED 212 which may be a multi-colored LED that can illuminate red, yellow, and green light. In an embodiment of this invention, an alarm signal my be used to warn a user that there is a low blood glucose reading, a partially filled blood glucose test strip, a low reservoir of insulin, an occlusion in insulin pump 300, a low battery status for insulin pump 300, a low battery status for remote controller 200, and/or an improperly filled test strip.

Blood glucose measurement means 220 may be a potentiostat designed for performing an episodic electrochemical measurement of a physiological fluid. In an embodiment of this invention, blood glucose measurement means 220 may apply a constant potential such as, for example, about +0.4 V between a working electrode and a reference electrode of a disposable test strip. A disposable test strip which may be suitable for use in the present invention is the OneTouch Ultra test strip which is commercially available from LifeScan, Inc. in Milpitas, California, U.S.A. The disposable test strip may be inserted into and electrically connected with strip port connector 214. After insertion, a physiological fluid such as blood may be applied to the disposable test strip causing the test to initiate. A reagent layer on the disposable test strip may proportionally convert an oxidized mediator to a reduced mediator allowing a current to be measured. A portion of the current may be sampled and mathematically converted to the glucose concentration which is displayed on display 202.

Port cover 209 in Figure 1 is an elastomeric material that covers over a wired connection port 224 and a memory chip port 232. Examples of a wired connection port may be a universal serial bus (USB) or a serial RS232. Wired connection port 224 may used to connect to a personal computer for transferring/receiving data and/or a software code.

Radio frequency (RF) transceiver 218 may be an electronic circuit capable of receiving and sending a wireless signal to a peripheral medical device. RF transceiver 218 may include a microprocessor for managing and processing a wireless signal. In an embodiment of this invention, RF transceiver 218 may use one of a variety of frequency formats and/or protocols such as, for example, an Industry-Scientific-Medical (ISM) frequency band, a Bluetooth format, a ZigBee format, and a WLAN format. In the ISM frequency band, the frequency may range from about 868 MHz to about 928 MHz. Antenna element 246 may be mounted to dielectric material 248 which in turn is mounted to bottom surface 256 of daughterboard 236. Antenna element 246 may be used as a conduit for sending and receiving wireless signals 310. Antenna impedance matching network 219 may be used to compensate for non-idealities or variations in the impedance of antenna element 246. Electrical signals corresponding to wireless signals 310 received by transceiver 218 are provided, following suitable processing, at connector 240 of daughterboard 236. Electrical signals corresponding to wireless signals 310 to be transmitted by transceiver 218 are provided by motherboard 234 at its connector 238.

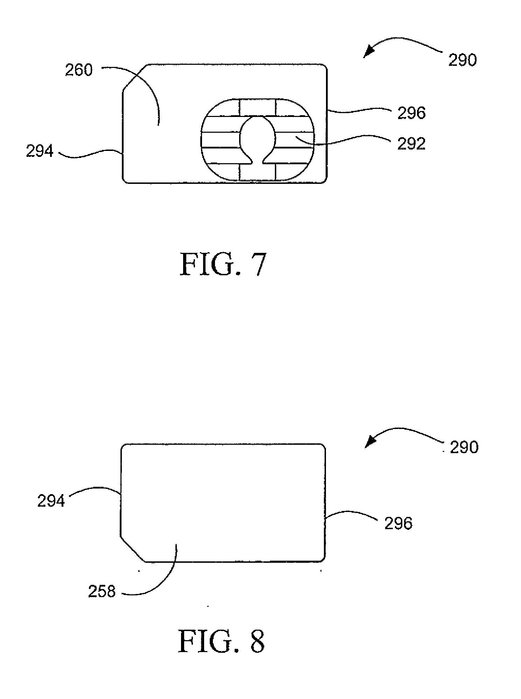

Figures 7 and 8 show a bottom and top plan view, respectively, of a memory chip 290 having a bottom surface 260 and a top surface 258. Memory chip 290 may be in the form of a non-volatile semiconductor memory such as, for example, a flash memory or an electrically erasable programmable read only memory (EEPROM). In an embodiment of this invention, memory chip 290 may be a type of flash memory called a Smart Card as shown in Figure 7 and 8.

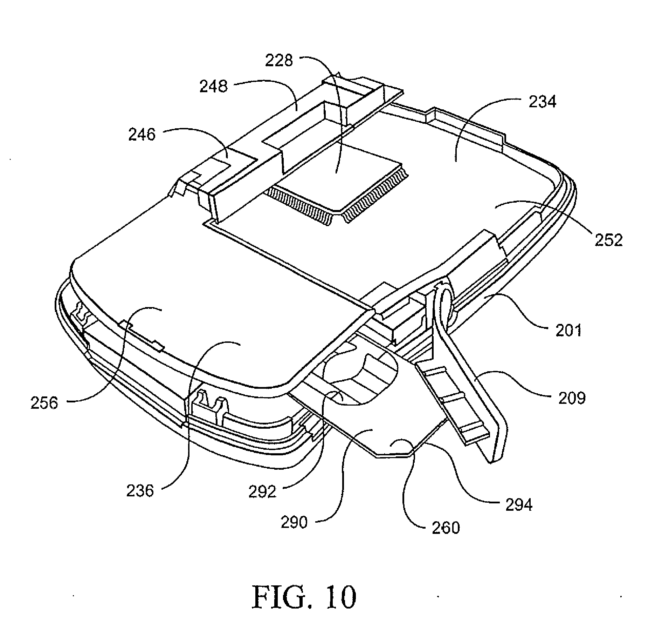

Figure 10 is a simplified schematic bottom perspective view of remote controller 200 with second housing half 207 removed. Memory chip 290 shown in Figures 7 and 8 may be inserted into memory chip port 232 for storing data and/or upgrading the software in remote controller 200, such as the software in microprocessor 228. Memory chip port 232 may be in the form of a slot on a side portion of remote controller 200 underneath port cover 209. Figure 10 shows a partial insertion of memory chip 290 into the slot. Memory chip port 232 is a connector having a plurality of memory chip port contacts 233 to electrically connect to a corresponding plurality of memory chip contacts 292 which are disposed on memory chip 290. Memory chip port 232 may be mounted on top surface 254 of daughterboard 236 allowing memory chip port contacts 233 to be facing upwards when inserted.

Memory chip 290 must be inserted into memory chip port 232 in a particular orientation such that a proper electrical connection can be established between memory chip 290 and remote controller 200. However, there are several orientations in which memory chip 290 may be inserted into memory chip port 232 and it may not be obvious to a user which way is proper. For example, there are four different permutations in which memory chip 290 may be inserted into memory chip port which are 1) top surface 258 facing upward towards a user and inserting initially a first end 294, 2) top surface 258 facing upward towards a user and inserting initially a second end 296, 3) bottom surface 260 facing upwards towards a user and inserting initially first end 294, and 4) bottom surface 260 facing upwards towards a user and inserting initially second end 296.

In an embodiment of this invention, memory chip 290 should have some printed artwork, such as an arrow and/or some text on either top surface 258 or bottom surface 260, to guide the user towards the proper orientation in inserting memory chip 290 and to clearly identify the memory chip 290. However, bottom surface 260 of memory chip 290 is substantially covered by memory chip contacts 292 making it difficult to print the artwork on bottom surface 260. Thus, the artwork should be printed on top surface 258 of memory chip 290. This will allow a user to view the artwork on top surface 258 of memory chip 290 while inserting it into memory chip port 232. In this configuration, memory chip contacts 292 should be facing downward and memory chip port contacts 233 should be facing upwards to allow for proper electrical connection.

It should be noted that daughterboard 236 provide an additional top surface 254 in which memory chip port 232 can be mounted such that memory chip port contacts 233 face upwards. It is not practical to mount memory chip port to top surface 250 of motherboard 234 because of a lack of usable space. As mentioned earlier, top surface 250 of motherboard 234 was predominantly occupied by navigational buttons 216 and display 202. Thus, the use of daughterboard 236 helps conserve space by allowing memory chip port 232 to mounted to it with memory chip connector contacts 233 facing upwards. This prevents having to mount memory chip port 232 to top surface 250 of motherboard 234 which would increase the size of motherboard 234 which is undesirable increase because this would, in turn, increase the size of remote controller 200.

It is an advantage of this invention that remote controller 200 can adapt or upgrade its wireless hardware in a simple and flexible manner. Because the wireless circuitry is compartmentalized from the blood glucose measuring circuitry, it is a simple procedure to replace the wireless circuitry which in this case is located on daughterboard 236. Thus, daughterboard 236 may be swapped with a new one having a different set of wireless components mounted thereon while retaining the circuitry located on the motherboard 234 for measuring glucose.

It is another advantage of this invention that a modular glucose meter having a wireless functionality can have a simpler to perform quality control procedure. Because the functional circuitry for the wireless capability and the glucose measurement are on separate circuit boards, separate quality control tests can be performed on the spatially separated circuit boards. The wireless signal generated by the wireless circuitry may interfere with the quality control testing for the glucose measuring circuitry. This would make the quality control procedure complicated to perform if both sets of circuits are electrically connected and in close proximity to each other. Thus, the likelihood of encountering interferences during quality control testing will be reduced by performing the two quality control tests separately such that motherboard 234 and daughterboard 236 are not in close proximity to each other.

It is yet another advantage of this invention that daughterboard 236 helps economizes space on motherboard 234. A sufficiently large LCD and navigation buttons can be mounted to top surface 250 of motherboard 234 that are easy for a user to see while at the same time allowing remote controller 200 to have a relatively small footprint. Further, daughterboard 236 allows memory chip port 232 to be mounted such that the memory chip 290 can be inserted with memory chip contacts 292 facing downward. This makes the insertion of memory chip 290 easier because the artwork can be placed on top surface 258 of memory chip 290 prompting and guiding the user to insert memory chip 290 with the proper orientation.

## Claims

1. A blood glucose meter (200) comprising:
a blood glucose measuring module (234) for performing a blood glucose measuring function and
a wireless communication module (236) adapted for establishing a wireless communication link with an external device and exchanging information with the external device via the wireless communication link according to a predetermined frequency format and protocol,
wherein the blood glucose measuring module (234) and the wireless communication module (236) are physically separate units electrically connected in order to allow for an exchange of electrical signals corresponding to information to be transmitted by the wireless communication module (236) and/or information received by the wireless communication module (236),
**characterized in that** the blood glucose measuring module (234) and the wireless communication module (236) both comprise a connector component (238, 240), wherein the two connector components (238, 240) are adapted to releasably establish the electrical connection between the blood glucose measuring module (234) and the wireless communication module (236), wherein the wireless communication module (236) comprises a memory chip connector (232) for electrically connecting the wireless communication module (236) with a non-volatile semi-conductor memory card (290).

2. The blood glucose meter according to claim 1, wherein the frequency format and/or protocol according to which the blood glucose meter (200) can communicate with an external device can be changed by replacing the wireless communication module (236) with another wireless communication module (236) which is adapted to communicate according to a different frequency format and/or protocol.

3. The blood glucose meter according to claim 1 or claim 2, wherein the blood glucose measuring module (234) comprises a control means (228) operable to control the electrical signals provided by the blood glucose measuring module (234) at its connector component (238).

4. The blood glucose meter according to any of the preceding claims, wherein the frequency format and/or protocol according to which the blood glucose meter (200) can communicate with an external device can be changed solely by replacing the wireless communication module (236) with another wireless communication module (236) which is adapted to communicate according to a different frequency format and/or protocol.

5. The blood glucose meter according to claim 3, wherein the wireless communication module (236) comprises a memory chip connector (232) for electrically connecting the wireless communication module (236) with a non-volatile semi-conductor memory card (290), and wherein the control means (228) of the blood glucose measuring module (234) is adapted to operate in accordance with information stored in a non-volatile semiconductor memory card (290) connected to the memory chip connector (232) of the wireless communication module (236).

6. The blood glucose meter according to claim 3, wherein the control means (228) can be reprogrammed in order to change the electrical signals provided during operation by the blood glucose measuring module (234) at its connector component (238).

7. The blood glucose meter according to claim 6, wherein the wireless communication module (236) comprises a memory chip connector (232) for electrically connecting the wireless communication module (236) with a non-volatile semi-conductor memory card (290), and wherein the control means (228) of the blood glucose measuring module (234) can be reprogrammed in accordance with information stored in a non-volatile semiconductor memory card (290) connected to the memory chip connector (232) of the wireless communication module (236).

8. The blood glucose meter according to claim 6 or claim 7, wherein the frequency format and/or protocol according to which the blood glucose meter (200) can communicate with an external device can be changed by replacing the wireless communication module (236) with another wireless communication module (236) which is adapted to communicate according to a different frequency format and/or protocol and reprogramming the control means (228) of the blood glucose measuring module (234).

9. The blood glucose meter according to any of the preceding claims, wherein the blood glucose measuring module (234) comprises a power supply for operating the wireless communication module (236), and wherein the electrical signals provided during operation by the blood glucose measuring module (234) at its connector component (238) include signals for supplying power to the wireless communication module (236).

10. The blood glucose meter according to any of the preceding claims, wherein the wireless communication module (236) comprises a transceiver (218), an antenna impedance matching network (21) and an antenna (246, 248).

11. The blood glucose meter according to any of the preceding claims, wherein the blood glucose measuring module (234) comprises a first printed circuit board on which blood glucose measuring circuitry (220) is mounted, and the wireless communication module (236) comprises a second printed circuit board on which wireless communication circuitry (218, 219, 246) is mounted.

12. The blood glucose meter according to claims 10 and 11, wherein the transceiver (218), the antenna impedance matching network (219) and the antenna (246, 248) are mounted on the second printed circuit board.

13. The blood glucose meter according to any of claims 11 to 12, wherein the first printed circuit board is a motherboard and the second printed circuit board is a daughterboard and/or wherein the second printed circuit board is disposed on the first printed circuit board.

14. The blood glucose meter according to any of the preceding claims, wherein one of the connector components (238, 240) of the blood glucose measuring module (234) and the wireless communication module (236) is the plug component of a plug and socket connector and the other connector component (238, 240) is the socket component of the plug and socket connection or wherein the connector components (238, 240) of the blood glucose measuring module (234) and the wireless communication module (236) are adapted to be connected by a cable having suitable connector components at its ends.

15. A blood glucose meter system comprising:
a blood glucose meter (200) according to any of claims 1 to 14, and
at least one further wireless communication module (236) similar to the wireless communication module (236) but adapted for establishing a wireless communication link with an external device and exchanging information with the external device via the wireless communication link according to a predetermined frequency format and protocol different from the frequency format and/or protocol of the wireless communication module (236),
wherein the frequency format and/or protocol according to which the blood glucose meter (200) can communicate with an external device can be changed by replacing the wireless communication module (236) with one of the at least one further wireless communication module (236).

16. The blood glucose meter system according to claim 15, wherein the wireless communication module (236) and the at least one further wireless communication module (236) include wireless communication modules (236) adapted to communicate according to the ISM, Bluetooth, ZigBee or WLAN standard.

17. A method of constructing a blood glucose meter (200) according to any of claims 1 to 14 comprising the steps of:
providing a blood glucose measuring module (234) for performing a blood glucose measuring function,
providing a plurality of wireless communication modules (236), each adapted for establishing a wireless communication link with an external device and exchanging information with the external device via the wireless communication link according to a predetermined frequency format and protocol different from the frequency format and/or protocol of the other wireless communication modules (236), wherein the blood glucose measuring module (234) and the wireless communication modules (236) are physically separate units and wherein the blood glucose measuring module (234) and each wireless communication module (236) comprise respective connector components (238, 240) by means of which a selected one of the wireless communication modules (236) may be releasably electrically connected to the blood glucose measuring module (234) in order to allow for an exchange of electrical signals corresponding to information to be transmitted by the respective wireless communication module (236) and/or information received by the respective wireless communication module (236),
selecting one of the plurality of wireless communication modules (236) in accordance with the desired frequency format and protocol, and
establishing an electrical connection between the selected wireless communication module (236) and the blood glucose measuring module (234) by means of the respective connector components (238, 240).
